# EUROPEAN PATENT APPLICATION

(11) **EP 4 233 712 A1**
(43) Date of publication of application: **30.08.2023**
(21) Application number: 23158587.8
(22) Date of filing: 24.02.2023
(51) Int. Cl.: A61B 5/11, A61B 5/00, A61G 7/015, A61G 7/018

(54) **MONITOR TO DETECT A PATIENT'S SLEEP STATE AND MOVEMENT**

(30) Priority: 28.02.2022 US 202263314526 P
(71) Applicant: Hill-Rom Services, Inc., Batesville, IN 47006-9167 (US)
(72) Inventor: CHRISTIE, John D., Batesville, 47006-9167 (US); RECEVEUR, Timothy J., Batesville, 47006-9167 (US); ZERHUSEN, Robert M., Batesville, 47006-9167 (US); MANN, Nicholas A., Batesville, 47006-9167 (US)
(74) Representative: Findlay, Alice Rosemary

(57) **Abstract**

A patient support apparatus comprises a plurality of load cells, a frame, a mattress, a plurality sensors, and a control system. The mattress includes a plurality of inflatable zones positioned on the frame, the mattress and frame cooperating to direct any patient load through the mattress and frame to the load cells. Each of the plurality of a sensors measures the pressure in a respective inflatable zone of the mattress. The control system includes a controller operable to receive a separate signal from each of the plurality of sensors, and a physiological sensor, process the signals to identify movement data and sleep data and to display the movement data and sleep date on a segmented screen on a user interface.

## Description

The present disclosure relates to the use of sensors of a patient support apparatus, such as a hospital bed, for example, to detect sleep states of a patient and characterize the sleep states. More specifically, the present disclosure is directed to a system or method for communicating the information concerning a patient's sleep state to a caregiver.

The use of load cells in patient support apparatuses, such as hospital beds, for example, to measure patient weight is known. Over time, approaches to using the information from the load cells to detect patient movement and to issue an alert or notification when the patient moves beyond a particular threshold have been developed. The use of load cells to make these determinations and inferences based on the motion or movement is limited by the potential for external influences, such as the addition of equipment to the frame supported on the scale. When this is done, the existing information regarding the position of the patient is compromised as the weight distribution is changed unexpectedly.

The pressure sensors used to measure air pressure in zones of an inflatable mattress are used to control the inflation pressure in the zones to control the interface pressure experienced by a patient supported on the mattress. However, because of transient effects and lack of precision, air pressure sensors associated with mattress zones are not regularly used to measure patient information. In addition, caregivers or visitors may intermittently apply pressure to the mattress, thereby changing air pressure measurements and the distribution of the weight on the frame. Motion algorithms generally rely on changes in the distribution of weight over multiple sensors to determine patient location and relative movement. These transient and external forces confound the algorithms used to determine patient movement and motion.

In some cases, it is important to determine patient movement relative to the patient support apparatus. Movement in this context means a change in position of the patient on the patient support apparatus, such as rolling over or moving toward an edge of a patient support apparatus to exit the patient support apparatus. Additionally, other sensors may be used to measure a patient's physiological characteristics such as heart rate or respiratory rate. A patient's mobility and a patient's physiological characteristics may be indicative of a patient's health and/or medical needs.

Monitoring the patient's sleep state may include monitoring for REM sleep and sleep Apnea. A caregiver may be able to respond to the patient based on the patient's sleep state and prevent additional apnea events. It may also be important that the caregiver is aware of the overall improvement or decline of a patient's health so they can assess how the patient's sleep activity related to the patient's overall health and direct the care the patient needs. Thus, the characterization of a patient's sleep information along with other physiological characteristics should be communicated in a manner that provides the required information to the caregiver and allows the caregiver to provide optimal care for the patient.

The present disclosure includes one or more of the following features, alone or in any combination.

According to a first aspect of the present disclosure, a patient support apparatus comprises a plurality of apparatus component sensors, a physiological sensor, a user interface including a segmented display, the segmented display illustrating movement data and sleep state data of a patient, and a control system including a controller in communication with the plurality of bed component sensors and the physiological sensor, the controller operable to receive a separate signal from each of the apparatus component sensors to monitor movement detected by the apparatus component sensors, a sleep signal from the physiological sensor, and wherein the controller is further operable to process the signals to determine the movement data and sleep data to detect a sleep event and alter a portion of the patient support apparatus to mitigate the sleep event.

In one embodiment, the control system comprises a controller, the controller operable to receive a separate signal from each of the apparatus component sensors to monitor patient movement detected by each of the apparatus component sensors, a heart rate and/or respiration rate signal from the physiological sensor. In another embodiment, the segmented display comprises a first segment including an icon for each day of a week, and wherein the activation of the icon for a particular day results in an illustration of the movement data or the sleep data for that day in a second segment.

In some embodiments, the controller moves a head section of the patient support apparatus in response to the sleep event. In some embodiments, the sleep event is a sleep apnea event.

In some embodiments, the patient support apparatus comprises a siderail having an illuminated grip and the sleep state of the patient is indicated by illuminating the grip using a specific scheme to indicate the sleep state.

In one embodiment, the second segment displays the movement data, and wherein the movement data comprises patient motion data and non-patient motion artifacts. In some embodiments, the second segment displays the movement data, and wherein the movement data comprises upper torso movement and lower body movement. In other embodiments, the second segment displays the movement data, and wherein the movement data comprises time the patient spent moving and not moving over a period of one week. In some embodiments, the second segment displays the movement data, and wherein the movement data comprises percentage of time the patient spent in upper torso movement and lower body movement over a period of one week.

In one embodiment, the second segment displays the sleep data, and wherein the sleep data comprises REM, light sleep state, or deep sleep state. In other embodiments, the second segment displays the sleep data, and the sleep data comprises time the patient spent sleeping, being in in bed or being out of bed. In some embodiments, the second segment displays the sleep data, and the sleep data comprises time the patient spent in REM, light sleep state, or deep sleep state over a period of one week. In some embodiments, the second segment displays the sleep data, and the sleep data comprises time the patient spent sleeping, being in in bed or being out of bed over a period of one week.

In one embodiment, the physiological sensor is operable to detect physiological characteristics of the patient. In some embodiments, the physiological characteristic is a heart rate. In some embodiments, the physiological characteristic is respiratory rate. In some embodiments, the physiological sensor provides both a heart rate and a respiratory rate signal. In some embodiment, the sleep status of the patient is projected on the floor.

According to a second aspect of the present disclosure, a system comprises a patient support surface including a plurality of inflatable zones, a plurality of load cells supporting the patient support surface, a plurality of air pressure sensors, each pressure sensor measuring the pressure in a respective inflatable zone of the patient support surface, a physiological sensor, and a controller operable to receive a separate signal from each of the plurality of load cells and each of the plurality of air pressure sensors, and a sleep signal from the physiological sensor, to process the signals to determine movement data and sleep data of a patient, and a user interface including a segmented display, the display illustrating the movement data and sleep data. The controller is further operable to process the signals to determine the movement data and sleep data to detect a sleep event and alter a portion of the patient support apparatus to mitigate the sleep event.

In one embodiment, the movement data comprises patient movement data and non-patient motion artifacts. In some embodiments, the patient movement data comprises upper torso movement and lower body movement. In other embodiments, the user interface displays the patient movement data and the time the patient spent moving and not moving. In some embodiments, the user interface displays percentage of time the patient spent in upper torso movement and lower body movement.

In one embodiment, the sleep data comprises REM, light sleep state, or deep sleep state. In some embodiments, the user interface displays the sleep data and time the patient spent in REM, light sleep state, or deep sleep state. In other embodiments, the user interface displays time the patient spent sleeping, being in in bed, and being out of bed.

In one embodiment, the physiological sensor is operable to detect physiological characteristics of the patient. In some embodiments, the physiological characteristic is a heart rate. In some embodiments, the physiological characteristic is respiratory rate. In some embodiments, the physiological sensor provides both a heart rate and a respiratory rate signal. In some embodiments, the sleep status of the patient is projected on the floor.

According to a third aspect of the present disclosure, a method of displaying movement data collected from a support apparatus comprising an inflatable mattress having multiple inflatable zones, the method comprises the steps of monitoring signals from a plurality of apparatus component sensors, positioning the mattress on a physiological sensor, monitoring sleep signals from the physiological sensor, using a controller to process the signals from the load cells, pressure sensors, and the physiological sensor to identify movement data and patient sleep data, and displaying the movement data and the sleep data of a patient on the mattress on a segmented display on a user interface.

In one embodiment, the method comprises automatically moving a portion of the support apparatus to mitigate a future event based on the movement data or the sleep data of the patient. In some embodiments, the future event is sleep apnea.

In one embodiment, the method comprises changing inflation of one of the multiple inflatable zones of the inflatable mattress based on the movement data or the sleep data of the patient. In some embodiments, the movement data comprises patient motion data and non-patient motion artifacts. In other embodiments, the movement data comprises upper torso movement and lower body movement. In one embodiment, the sleep data comprises REM, light sleep state, or deep sleep state. In some embodiments,

In one embodiment, the method comprises the physiological sensor detecting physiological characteristics of the patient. In some embodiments, the physiological characteristic is a heart rate. In some embodiments, the physiological characteristic is respiratory rate. In some embodiments, the physiological sensor provides both a heart rate and a respiratory rate signal. In some embodiments, the method comprises projecting the sleep status of the patient on the floor.

The invention will now be further described by way of example with reference to the accompanying drawings, in which:
Fig. 1 is a perspective view of a patient support apparatus including a control system operable to measure signals from a plurality of sensors and process those signals according to the present disclosure;
Fig. 2 is a block diagram of a portion of the control system of the patient support apparatus of Fig. 1;
Fig. 3 is a diagrammatic illustration of the interaction between a first frame of the patient support apparatus of Fig. 1 and a second frame supported on load cells supported from the first frame;
Fig. 4, is a side view of a portion of the patient support apparatus of Fig. 1 showing a first frame supported on load cells supported on a second frame, the load cells supporting all of the load of the first frame;
Fig. 5 is an illustration of one embodiment of a patient support associated with a physiological sensor that detects a patient's physiological characteristic;
Fig. 6 is an illustration of one embodiment of a user interface displaying a patient's mobility information;
Fig. 7 is an illustration of one embodiment of a display screen including a home icon, a rest icon, a sleep data icon, a movement icon, and a help icon, where the sleep data icon is activated;
Fig. 8 is an illustration of one embodiment of a sleep data screen including a sleep class icon and a sleep time icon;
Fig. 9 is an illustration of one embodiment of a sleep class screen shown upon activation of the sleep class;
Fig. 10 is an illustration of one embodiment of a sleep class history screen upon activation of the sleep class history icon;
Fig. 11 is an illustration of one embodiment of a sleep time screen upon activation of the sleep time screen icon;
Fig. 12 is an illustration of one embodiment of a sleep time history screen upon activation of the sleep time history icon;
Fig. 13 is an illustration of one embodiment of a display screen including a home icon, a rest icon, a sleep data icon, a movement icon, and a help icon, where the movement icon is activated;
Fig. 14 is an illustration of one embodiment of a movement screen including a movement data icon and a movement time icon;
Fig. 15 is an illustration of one embodiment of a movement data screen upon activation of the movement data icon;
Fig. 16 is an illustration of one embodiment of a movement data history screen upon activation of the movement data history icon;
Fig. 17 is an illustration of one embodiment of a movement time screen upon activation of the movement time icon; and
Fig. 18 is an illustration of one embodiment of a movement time history screen upon activation of the movement time history icon.

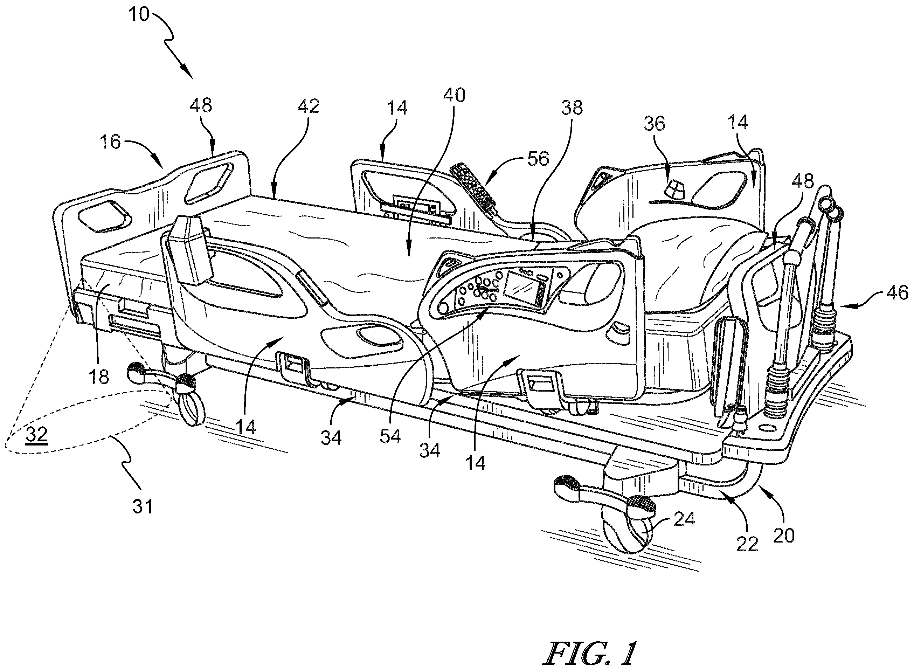

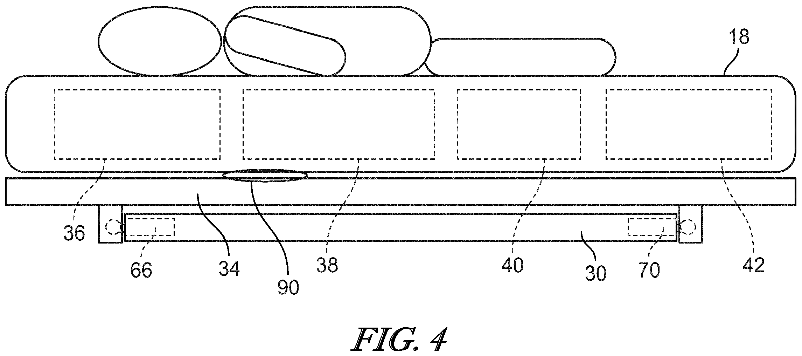

An illustrative patient support apparatus 10 embodied as a hospital bed is shown in Fig. 1. The bed 10 of Fig. 1 has a frame 20 which includes a base frame 22 supported on casters 24. The stationary base frame 22 further supports a weigh frame 30 that supports an adjustably positionable mattress support upper frame 34 supporting a mattress 18. As shown in Fig. 4, the illustrative mattress 18 is an inflatable patient support surface which includes inflatable zones including a head zone 36, a seat zone 38, thigh zone 40, and a foot zone 42. The bed 10 further includes a headboard 12 at a head end 46 of the bed 10, a footboard 16 at a foot end 48 of the bed 10, and a movable siderails 14 coupled to the upper frame 34 of the bed 10. The bed 10 also includes a user interface 54 positioned on one of the siderails 14. The bed 10 of the embodiment of Fig. 1 is conventionally configured to adjustably position the upper frame 34 relative to the base frame 22 to adjust the position of a patient supported on the mattress 18.

Conventional structures and devices are provided to adjustably position the upper frame 34, and such conventional structures and devices may include, for example, linkages, drives, and other movement members and devices coupled between base frame 22 and the weigh frame 30, and/or between weigh frame 30 and upper frame 34. Control of the position of the upper frame 34 and mattress 18 relative to the base frame 22 or weigh frame 30 is controlled, for example, by a patient control pendant 56 or user interface 54. The upper frame 34 may, for example, be adjustably positioned in a general incline from the head end 46 to the foot end 48 or vice versa. Additionally, the upper frame 34 may be adjustably positioned such that the head section 44 of the mattress 18 is positioned between minimum and maximum incline angles, e.g., 0-65 degrees, relative to horizontal or bed flat, and the upper frame 34 may also be adjustably positioned such that a seat section (not shown) of the mattress 18 is positioned between minimum and maximum bend angles, e.g., 0-35 degrees, relative to horizontal or bed flat. Those skilled in the art will recognize that the upper frame 34 or portions thereof may be adjustably positioned in other orientations, and such other orientations are contemplated by this disclosure.

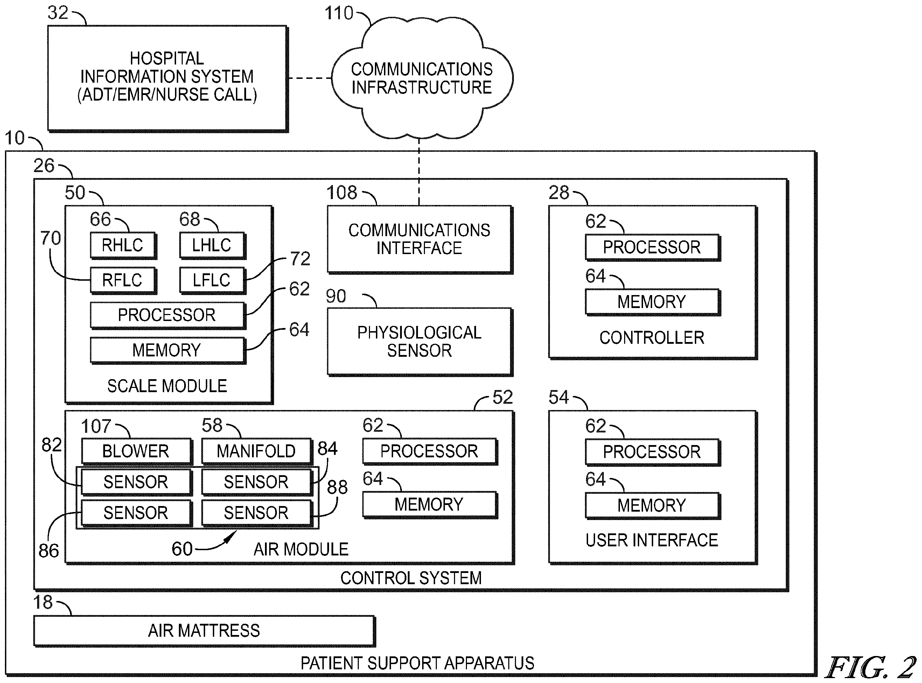

In one illustrative embodiment shown diagrammatically in Fig. 2, the bed 10 has a control system 26 that includes a controller 28, a scale module 50, an air module 52, and the user interface 54. In the illustrative embodiment each of the controller 28, scale module 50, air module 52, and user interface 54 includes a processor 62 and a memory device 64. The processor 62 and memory device 64 are shown only with respect to the controller 28, but similar structures are used in the scale module 50, air module 52, and user interface 54. The memory device 64 includes instructions that, when executed by the processor 62, causes the processor 62 to perform functions as associated with the particular one of controller 28, scale module 50, air module 52, and user interface 54. The components of the control system 26 communicate amongst themselves to share information and distribute the functions of the bed 10. The processor 62 of each of the controller 28, scale module 50, air module 52, and user interface 54 is also operable, based on instructions from the memory device 64, to communicate with the others of the controller 28, scale module 50, air module 52, and user interface 54 using a communications protocol. It should be understood that the term processor here includes any microprocessor, microcontroller, processor circuitry, control circuitry, preprogrammed device, or any structure capable of accessing the memory device and executing non-transient instructions to perform the tasks, algorithm, and processed disclosed herein. In the illustrative embodiment, the control system 26 employs a conventional controller area network (CAN) for communications between subsystems, but it should be understood that any of a number of networking and communications solutions may be employed in the control system 26.

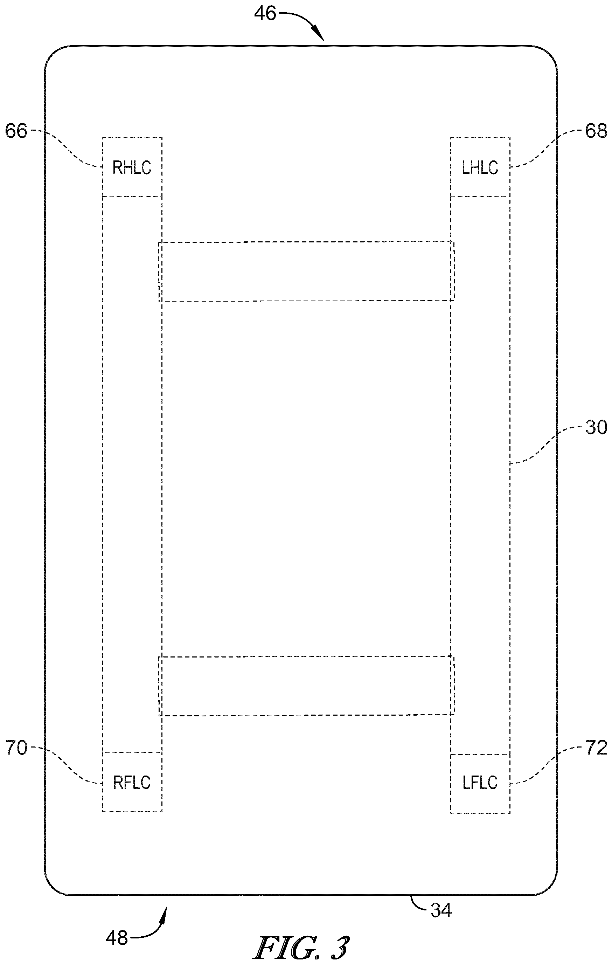

As shown in Fig. 3, the scale module 50 includes four load cells 66, 68, 70, and 72. To determine a weight of a patient supported on the mattress 18, the load cells 66, 68, 70, and 72 are positioned between the weigh frame 30 and the upper frame 34 as illustrated in Figs. 3 and 4. Each load cell 66, 68, 70, 72 is configured to produce a signal indicative of a load supported by the respective load cell 66, 68, 70, 72 from the upper frame 34 relative to the weigh frame 30. Some of the structural components of the bed 10 will be designated hereinafter as "right", "left", "head" and "foot" from the reference point of an individual lying on the individual's back on the mattress 18 with the individual's head oriented toward the head end 46 of the bed 10 and the individual's feet oriented toward the foot end 48 of the bed 10. Following this convention, the load cell 66 is designated as the right head load cell (RHLC) in the figures to represent that the load cell 66 is positioned at the right side of the bed 10 at the head end 46. The load cell 68 is designated at the left head load cell (LHLC), the load cell 70 is designated as the right foot load cell (RFLC), and the load cell 72 is designated left foot load cell (LFLC), each following the same convention.

The scale module 50 includes the processor 62 that is in communication with each of the respective load cells 66, 68, 70, and 72 and operable to process the signals from the load cells 66, 68, 70, and 72. The memory device 64 is also utilized by the controller 28 to store information corresponding to features and functions provided by the bed 10.

A weight distribution of a load among the plurality of load cells 66, 68, 70, and 72 may not be the same depending on variations in the structure of the bed 10, variations in each of load cells 66, 68, 70, and 72 and the position of the load on the mattress 18 relative to the particular load cell 66, 68, 70, or 72. Accordingly, a calibration constant for each of the load cells 66, 68, 70, and 72 is established to adjust for differences in the load cells 66, 68, 70, and 72 in response to the load borne by each. Each of the load cells 66, 68, 70, and 72 produces a signal indicative of the load supported by that load cell 66, 68, 70, or 72. The loads detected by each of the respective load cells 66, 68, 70, 72 are adjusted using a corresponding calibration constant for the respective load cell 66, 68, 70, 72. The adjusted loads are then combined to establish the actual weight supported on the bed 10. In the present disclosure, the independent signals from each of the load cells 66, 68, 70, 72 is used to draw inferences about the movement and motion of the patient.

The air module 52 is the functional controller for the mattress 18 and includes processor 62 and a memory device 64. The processor 62 is in communication with a blower 107, a manifold 58, and an air pressure sensor assembly 60. The air module 52 is a conventional structure with the manifold 58 operating under the control of the processor 62 to control the flow of air from the blower 107 into and out of the head zone 36, seat zone 38, thigh zone 40, and foot zone 42 to control the interface pressure experienced by the patient supported on the mattress 18. The sensor assembly 60 includes separate sensors for measuring the air pressure in each of the head zone 36, seat zone 38, thigh zone 40, and foot zone 42. The pressure sensor assembly includes a head zone sensor 82, a seat zone sensor 84, a thigh zone senor 86, and a foot zone sensor 88. While signals from the sensors 82, 84, 86, and 88 are used to control the pressure in the respective zones, applying the principles of the present disclosure, the signals are also useful in making inferences regarding patient movement and, when used synergistically with the information gleaned from the signals from the load cells 66, 68, 70, and 72, provide a more fulsome and accurate analysis of patient movement and/or any motion associated with the patient support apparatus.

The scale module 50 and air module 52 of the bed 10 are used for measuring the motions of a patient that occupies the bed 10. Referring to Fig. 4, a diagrammatic side view of a patient supported on the mattress 18 and frame 34 with the load of the patient being borne by the inflated zones 36, 38, 40, and 42 and passed through the mattress to the frame 34 to the load cells 66, 68, 70, and 72 supported from the weigh frame 30. As seen in Fig. 4, in a static condition, the patient's weight is appropriately distributed over the inflated zones 36, 38, 40, and 42. Each of those zones 36, 38, 40, and 42 are inflated to a target pressure based on the patient's weight detected by the scale module 50 and the expected distribution of the patient.

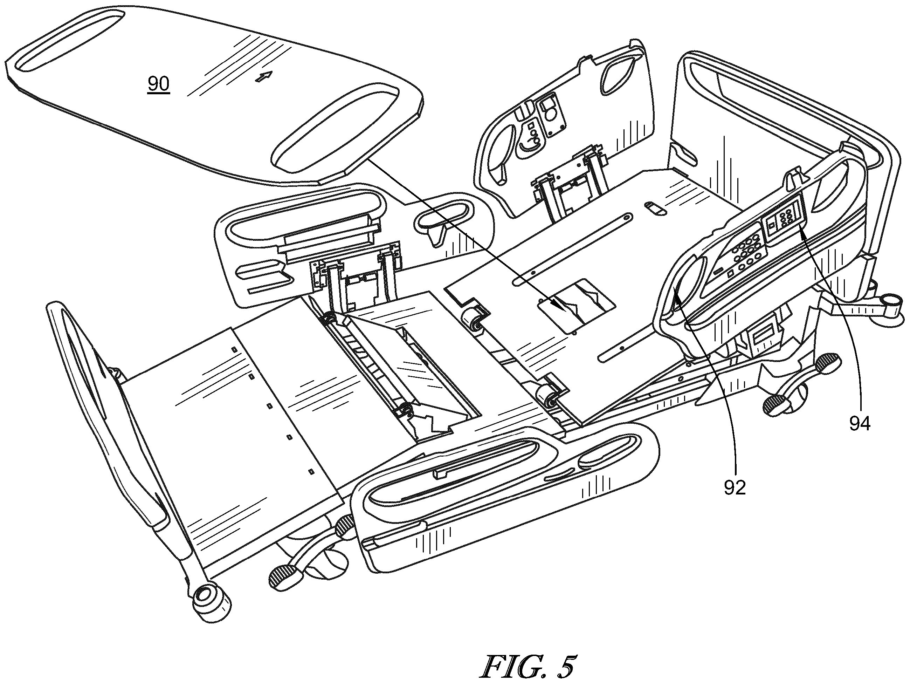

The bed 10 is also associated with a physiological sensor 90 that detects a patient's physiological characteristic such as heart rate and respiratory rate. As shown in Fig. 5, the physiological sensor 90 is be located on the bed 10. The physiological sensor 90 extrapolate the patient's sleep state from detectable data such as heart rate and respiratory rate. If the physiological sensor 90 detects REM sleep, the information is displayed on the user interface 54 including an illuminated grip 92 on the siderails 14 of the bed 10. U.S. Pat. No. 11,172,892, issued November 16, 2021 and titled "PATIENT SUPPORT APPARATUS HAVING VITAL SIGNS MONITORING AND ALERTING" (the `892 patent) discloses a scheme of providing various indicators of patient states at the user interface 54 and illuminated grip 92. The indications disclosed herein are contemplated to be in addition to that scheme and may use a different color, other than those already disclosed in the `892 patent, such as magenta, to provide an indication of a sleep state. If REM sleep is detected, the grip 92 displays the magenta color indicating to caregivers the patient's sleep state. In other embodiments, the information may also or alternatively be displayed though other means such as text on the user interface. Since REM sleep is vital to a patient's recovery, caregivers can avoid interrupting this sleep cycle and plan patient care accordingly. If sleep apnea events are detected, the illuminated grip 92 will display a flashing magenta color indicator and/or send an alert to caregivers, such as by a text message, for example.

In some embodiments, when the physiological sensor 90 detects sleep apnea events, the controller 28 automatically raises a portion of the bed 10 a few degrees to prevent future occurrence of sleep apnea. Control circuitry receives user input commands from the physiological sensor 90. The processor 62 uses the information to control various functions of bed 10 including the movement of the head zone 36. In some embodiments, the bed 10 automatically raises the head zone 36, the seat zone 38, the thigh zone 40, or the foot zone 42 in response to signals from the physiological sensor 90 or bed component sensors such as load cells 66, 68, 70, and 72 and/or air pressure sensor assembly 60. The inflation in the head zone 36, the seat zone 38, the thigh zone 40, or the foot zone 42 of the mattress 18 can be varied based on signals from the physiological sensor 90 or bed component sensors such as load cells 66, 68, 70, and 72 and/or air pressure sensor assembly 60.

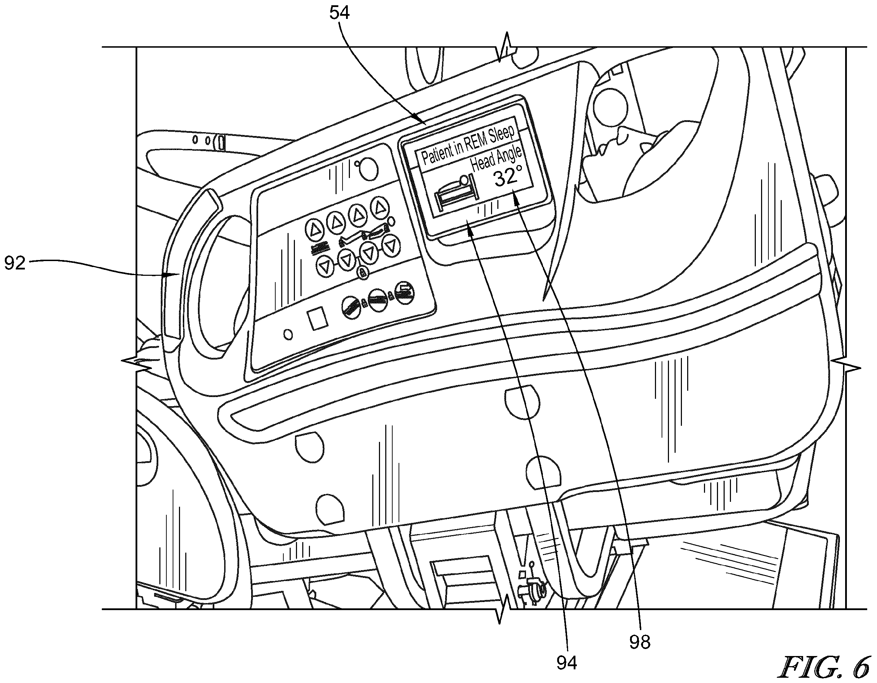

As shown in Fig. 6, the sleep apnea event is indicated on the display screen 94 and on the illuminated grip 92 of the user interface 54. As shown in the embodiment of Fig. 6, the sleep apnea event is indicated as a flashing color indication. In some embodiments, the bed 10 logs the sleep status and sends data to a central care station or electronic medical records system ("EMR").

In some embodiments, the sleep state of the patient on the bed 10 is be indicated on the user interface 54. The display screen 94 includes a status board 98 indicating that the sleep state of the patient (e.g., if the patient is in REM sleep) as well as a head angle of the patient on the bed 10. This sleep status board 98 also helps caregivers track the head angle of the patient with respect to sleep apnea or medically induced sleep apnea.

The data indicating patient weigh and/or patient movement as determined by the bed component sensors such as load cells 66, 68, 70, and 72 and/or air pressure sensor assembly 60 may be combined with data from physiological sensor 90 to provide a more detailed record of patient sleep. The discerned data may be displayed in detail on the display screen 94 of the user interface 54 on the siderail 14 of the bed 10. The data may include different sleep and movement states of the patient. The sleep status of the patient may also be communicated by icons illuminated on the bed 10 or as icons projected on the floor. For example, as shown in Fig. 1, the REM sleep indicator icon 31 represented by the dotted projection 31 shown in Fig. 1 may be projected on the floor 32. The illuminated icons and projected icon 31 are used to indicate that the patient is receiving the optimal sleep for all sleep indicators being monitored. The illuminated icons or projected icon 31 may be a particular color associated with sleep, such as the magenta color discussed above, for example. In addition, a particular iconic representation associated with a sleep state may be used for each sleep state or a particular sleep event.

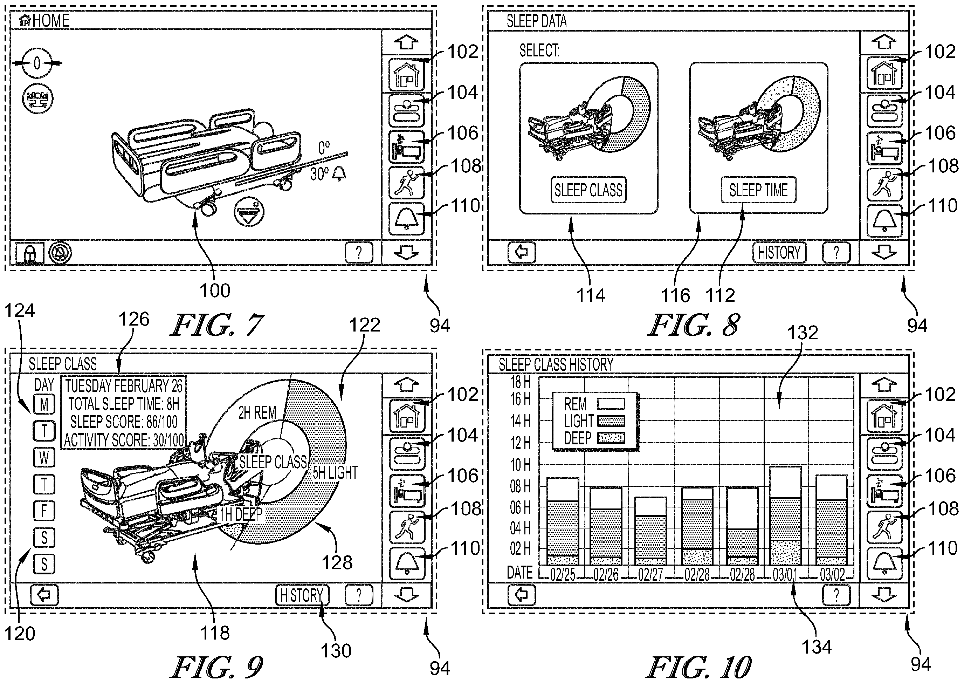

The display screen 94 of the user interface 54 may include more than one input icons. As show in Fig. 7, the home screen 100 on the display screen 94 includes a home icon 102, a rest icon 104, a sleep data icon 106, a movement icon 108, and a help icon 110. The controller 28 receives user input commands from graphical display screen 94 when any icon is activated. Based on the user input commands on the display 94 and the data processed by the processor 62, the controller 28 controls various functions of bed 10 such as the pneumatic system and/or raising or lowering different components of the bed 10. In some embodiments, based on the user input commands on the display 94 and the data processed by the processor 62, the controller 28 controls the functions of one or more of actuators in the bed 10. In some based on the user input commands on the display 94 and the data processed by the processor 62, the controller 28 controls functions of the load cells 66, 68, 70, and 72 and/or air pressure sensor assembly 60.

As shown in Fig. 8, when a caregiver activates the sleep data icon 106, the controller 28 prompts the caregiver to select either the sleep class icon 114 or the sleep time icon 112 on the sleep data screen 116. As shown in Fig. 9, the sleep class icon 114 is activated by a caregiver to cause the controller 28 to call up a sleep class screen 118. The sleep class screen 118 of the user interface 54 is a segmented display screen 94 including one or more segments. The one or more segments in the display screen 94 are one or more portions in the display screen 94 showing different information. The first segment 120 includes a plurality of icons 124, each for a day of the week. One of the plurality of icons 124 may be activated by a caregiver to cause the controller 28 to display the patient's sleep data 126, 128 on the second segment 122. The patient's sleep data 126, 128 includes the time the patient spent in REM sleep, light sleep, or deep sleep on the day selected from the plurality of icons 124 on the first segment 120. The sleep data 128 is shown to be segmented with different portions of the chart indicating different sleep states. In some embodiments, the variations in stippling in the chart represented at reference 128 may be different shades of a particular color, such as magenta, for example.

The sleep class screen 118 also includes a sleep class history icon 130. As shown in Fig. 10, the sleep class history icon 130 may be activated by a caregiver to cause the controller 28 to display the patient's sleep class history on a sleep class history screen 132. The sleep class history screen 132 may include data showing the duration or frequency of REM sleep, light sleep, or deep sleep over a fixed time period (e.g., a week) 134. The sleep data 134 is shown to be segmented with different portions of the chart indicating different sleep states. In some embodiments, the variations in stippling in the chart represented at reference 134 may be different shades of a particular color, such as magenta, for example.

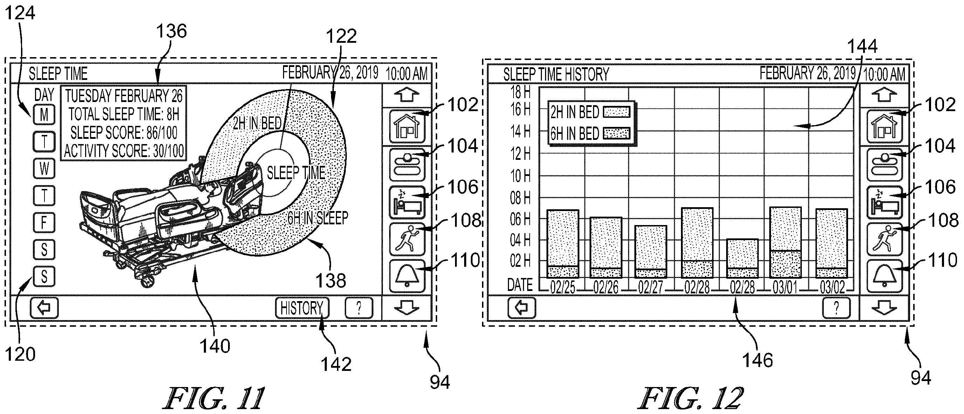

As show in Fig. 11, the sleep time icon 112 shown in Fig, 8 may be activated by a caregiver to cause the controller 28 to call up a sleep time screen 140. The first segment 120 of the sleep time screen 140 includes a plurality of icons 124, each for a day of the week. One of the plurality of icons 124 may be activated by a caregiver to cause the controller 28 to display the patient's sleep data 136, 138 in the second segment 122. The patient's sleep data 136, 138 includes the time the patient spent in bed and the time the patient spent sleeping on the day selected from the plurality of icons 124 on the first segment 120. The sleep data 138 is shown to be segmented with different portions of the chart indicating different sleep states. In some embodiments, the variations in stippling in the chart represented at reference 138 may be different shades of a particular color, such as magenta, for example.

The sleep time screen 140 includes a sleep time history icon 142. As shown in Fig. 12, the sleep time history icon 142 may be activated by a caregiver to cause the controller 28 to display the patient's sleep time history on a sleep time history screen 144. The sleep time history screen 144 may include data showing the patient spent in bed and the time the patient spent sleeping over a fixed time period (e.g., a week) 146. The sleep data 146 is shown to be segmented with different portions of the chart indicating different sleep states. In some embodiments, the variations in stippling in the chart represented at reference 146 may different shades of a particular color, such as magenta, for example.

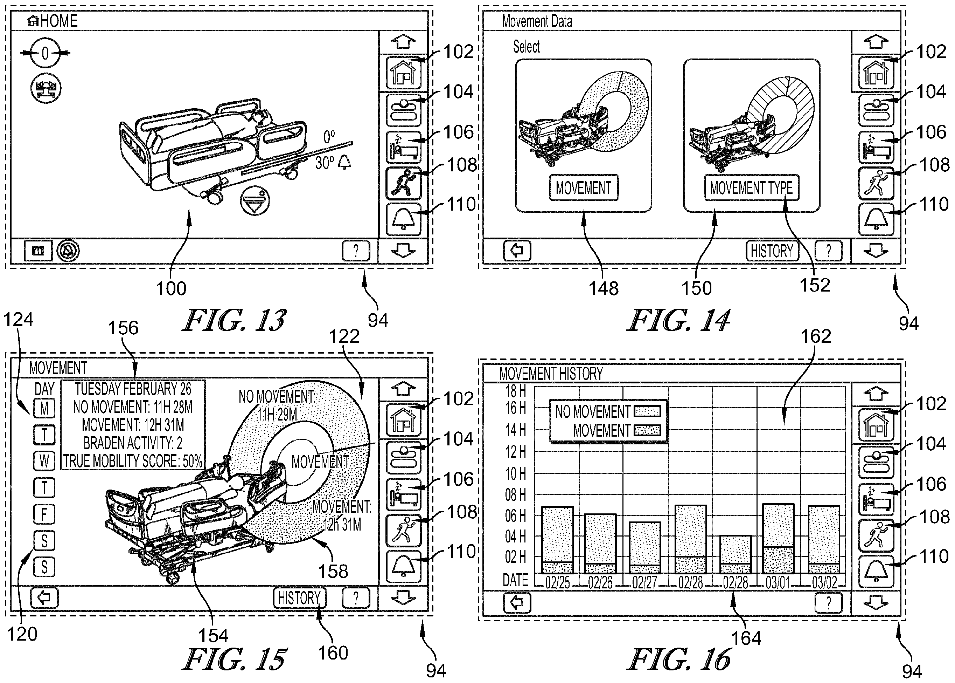

As show in Fig. 13, the home screen 100 on the display screen 94 includes a movement icon 108, which may be activated by a caregiver. As show in Fig. 14, when a caregiver activates the movement icon 108, the controller 28 prompts the caregiver to select either the movement data icon 148 or the movement type icon 152 on the movement screen 150. As show in Fig. 15, the movement data icon 148 may be activated by a caregiver to cause the controller 28 to call up a movement data screen 154. The first segment 120 includes a plurality of icons 124, each for a day of the week. One of the plurality of icons 124 may be activated by a caregiver to cause the controller 28 to display the patient's sleep data 156, 158 on the second segment 122. The patient's movement data 156, 158 includes the time the patient spent in motion and the time the patient spent not moving or being subject to non-motion artifacts on the day selected from the plurality of icons 124 in the first segment 120. The sleep data 158 is shown to be segmented with different portions of the chart indicating different sleep states. In some embodiments, the variations in stippling in the chart represented at reference 158 may be different shades of a particular color, such as magenta, for example.

The movement data screen 154 includes a movement data history icon 160. As show in Fig. 16, the movement data history icon 160 may be activated by a caregiver to cause the controller 28 to display the patient's movement data history on a movement data history screen 162 over a fixed time period (e.g., a week) 164. The sleep data 164 is shown to be segmented with different portions of the chart indicating different sleep states. In some embodiments, the variations in stippling in the chart represented at reference 164 may different shades of a particular color, such as magenta, for example.

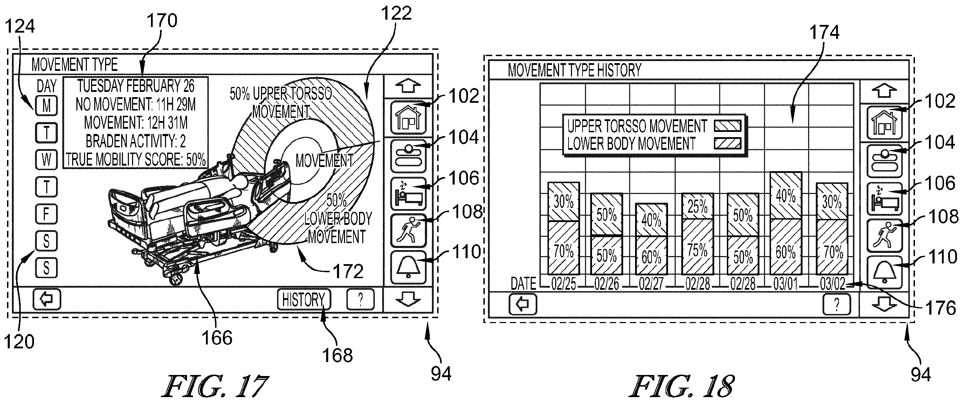

As show in Fig. 17, the movement type icon 152 shown in Fig. 14, may be activated by a caregiver to cause the controller 28 to call up a movement time screen 166. The first segment 120 of the sleep time screen 166 includes a plurality of icons 124, each for a day of the week. One of the plurality of icons 124 may be activated by a caregiver to cause the controller 28 to display the patient's movement data 170, 172 in the second segment 122. The patient's movement data 170, 172 includes the percentage of patient movement comprising movement types such as upper torso movement and lower body movement on the day selected from the plurality of icons 124 on the first segment 120.

The movement time screen 166 includes a movement time history icon 168. As show in Fig. 18, the movement type history icon 168 may be activated by a caregiver to cause the controller 28 to display the patient's movement time history on a movement type history screen 174. The movement type history screen 174 may include data showing movement types such as upper torso movement and lower body movement over a fixed time period (e.g., a week) 176.

Although this disclosure refers to specific embodiments, it will be understood by those skilled in the art that various changes in form and detail may be made without departing from the subject matter set forth in the accompanying claims.

Embodiments of the invention can be described with reference to the following numbered clauses, with preferred features laid out in the dependent clauses:
1. A system comprising:
   a patient support surface including a plurality of inflatable zones,
   a plurality of load cells supporting the patient support surface,
   a plurality of air pressure sensors, each pressure sensor measuring the pressure in a respective inflatable zone of the patient support surface,
   a physiological sensor, and
   a controller operable to receive a separate signal from each of the plurality of load cells and each of the plurality of air pressure sensors, and a sleep signal from the physiological sensor, to process the signals to determine movement data and sleep data of a patient, and
   a user interface including a segmented display, the display illustrating the movement data and sleep data, and
      wherein the controller is further operable to process the signals to determine the movement data and sleep data to detect a sleep event and alter a portion of the patient support apparatus to mitigate the sleep event.
2. The system of clause 1, wherein the movement data comprises patient movement data and non-patient motion artifacts.
3. The system of either clause 1 or clause 2, wherein the patient movement data comprises upper torso movement and lower body movement.
4. The system of any preceding clause, wherein the user interface displays the patient movement data and the time the patient spent moving and not moving.
5. The system of clause 4, wherein the user interface displays percentage of time the patient spent in upper torso movement and lower body movement.
6. The system of any preceding clause, wherein the physiological sensor is operable to detect a respiration rate and heart rate of the patient.
7. The system of clause 6, wherein the sleep data comprises REM, light sleep state, or deep sleep state.
8. The system of clause 7, wherein the user interface displays the sleep data and time the patient spent in REM, light sleep state, or deep sleep state.
9. The system of either clause 7 or clause 8, wherein the user interface displays time the patient spent sleeping, being in in bed, and being out of bed.
10. The system of any preceding clause, wherein the sleep status of the patient is projected on the floor.
11. The system of any preceding clause, wherein the sleep event comprises a sleep apnea event.
12. The system of clause 11, wherein the patient support apparatus comprises a movable head section and the controller raises the head section to mitigate the sleep apnea.
13. The system of either clause 11 or clause 12, wherein the patient support apparatus comprises a movable head section and the controller raises the head section to mitigate the sleep apnea.
14. A method of displaying movement data collected from a support apparatus comprising an inflatable mattress having multiple inflatable zones, the method comprising the steps of:
   monitoring signals from a plurality of load cells supporting a patient support deck of the patient support apparatus,
   positioning the mattress on a physiological sensor,
   monitoring signals from the physiological sensor,
   using a controller to process the signals from the load cells, pressure sensors, and the physiological sensor to identify movement data and patient sleep data, and
   displaying the movement data and the sleep data of a patient on the mattress on a segmented display on a user interface.
15. The method of clause 14, wherein the method comprises automatically moving a portion of the support apparatus to mitigate a future event based on the movement data or the sleep data of the patient.
16. The method of clause 15, wherein the future event is sleep apnea.
17. The method of any one of clauses 14 to 16, wherein the method comprises changing inflation of one of the multiple inflatable zones of the inflatable mattress based on the movement data or the sleep data of the patient.
18. The method of any one of clauses 14 to 17, wherein the movement data comprises patient motion data and non-patient motion artifacts.
19. The method of any one of clauses 14 to 18, wherein the movement data comprises upper torso movement and lower body movement.
20. The method of any one of clauses 14 to 19, wherein the physiological sensor is operable to detect physiological characteristics of the patient.
21. The method of any one of clauses 14 to 20, wherein the sleep data comprises REM, light sleep state, or deep sleep state.
22. The method of any one of clauses 14 to 21, wherein the sleep status of the patient is projected on the floor.
23. A patient support apparatus comprising:
   a patient support surface;
   a siderail located adjacent the patient support surface;
   a headboard located adjacent the patient support surface;
   a footboard located adjacent the patient support surface;
   a frame supporting the patent support surface;
   at least one sensor supported by the frame and located lower than the patient support surface, wherein the sensor is operable to generate, without contacting the patient, signals indicative of at least one of a heart rate, respiration rate, and movement of a patient supported on the support surface;
   a controller operable to process the signals to determine at least one of movement data and sleep data of a patient;
   a user interface held to at least one of the siderail, headboard, footboard, frame, and sensor, the user interface illustrating at least one of a movement condition generated from the movement data and a sleep condition generated from the sleep data.
24. The patient support apparatus as recited in clause 22, wherein the user interface comprises at least one of an indicator light and a projection light.
25. The patient support apparatus as recited in either clause 22 or clause 23, wherein the user interface comprises a touchscreen display held to at least one of the siderail, headboard, and footboard.
26. The patient support apparatus as recited in any one of clauses 22 to 24, wherein the user interface comprises an integrated light held within at least one of the siderail, headboard, footboard, and frame.

Further embodiments of the invention can be described with reference to the following numbered clauses, with preferred features laid out in the dependent clauses:
1. A patient support apparatus comprising:
   a plurality of apparatus component sensors,
   a physiological sensor,
   a control system including a controller in communication with the plurality of bed component sensors and the physiological sensor, and
   a user interface including a segmented display, the segmented display illustrating movement data and sleep state data of a patient.
2. The patient support apparatus of clause 1, wherein the control system comprises a controller, the controller operable to receive a separate signal from each of the apparatus component sensors to monitor energy detected by each of the apparatus component sensors, a sleep signal from the physiological sensor, and the controller is further operable to process the signals to determine the movement data and sleep data.
3. The patient support apparatus of either clause 1 or clause 2, wherein the segmented display comprises a first segment including an icon for each day of a week, and wherein the activation of the icon for a particular day results in an illustration of the movement data or the sleep data in a second segment.
4. The patient support apparatus of clause 3, wherein the second segment displays the movement data, and wherein the movement data comprises patient motion data and non-patient motion artifacts.
5. The patient support apparatus of either clause 3 or clause 4, wherein the second segment displays the movement data, and wherein the movement data comprises upper torso movement and lower body movement.
6. The patient support apparatus of any one of clauses 3 to 5, wherein the second segment displays the movement data, and wherein the movement data comprises time the patient spent moving and not moving over a period of one week.
7. The patient support apparatus of and one of clauses 3 to 6, wherein the second segment displays the movement data, and wherein the movement data comprises percentage of time the patient spent in upper torso movement and lower body movement over a period of one week.
8. The patient support apparatus of any one of clauses 3 to 7, wherein the second segment displays the sleep data, and wherein the sleep data comprises REM, light sleep state, or deep sleep state.
9. The patient support apparatus of any one of clauses 3 to 8, wherein the second segment displays the sleep data, and wherein the sleep data comprises time the patient spent sleeping, being in in bed or being out of bed.
10. The patient support apparatus of any one of clauses 3 to 9, wherein the second segment displays the sleep data, and wherein the sleep data comprises time the patient spent in REM, light sleep state, or deep sleep state over a period of one week.
11. The patient support apparatus of any one of clauses 3 to 10, wherein the second segment displays the sleep data, and wherein the sleep data comprises time the patient spent sleeping, being in in bed or being out of bed over a period of one week.
12. The patient support apparatus of any preceding clause, wherein the physiological sensor is operable to detect physiological characteristics of the patient.
13. The patient support apparatus of any preceding clause, wherein the sleep status of the patient is projected on the floor.
14. A system comprising:
   a patient support surface including a plurality of inflatable zones,
   a plurality of load cells supporting the patient support surface,
   a plurality of air pressure sensors, each pressure sensor measuring the pressure in a respective inflatable zone of the patient support surface,
   a physiological sensor, and
   a controller operable to receive a separate signal from each of the plurality of load cells and each of the plurality of air pressure sensors, and a sleep signal from the physiological sensor, to process the signals to determine movement data and sleep data of a patient, and
   a user interface including a segmented display, the display illustrating the movement data and sleep data.
15. The system of clause 14, wherein the movement data comprises patient movement data and non-patient motion artifacts.
16. The system of clause 15, wherein the patient movement data comprises upper torso movement and lower body movement.
17. The system of clause 16, wherein the user interface displays the patient movement data and the time the patient spent moving and not moving.
18. The system of clause 17, wherein the user interface displays percentage of time the patient spent in upper torso movement and lower body movement.
19. The system of any one of clauses 14 to 18, wherein the physiological sensor is operable to detect physiological characteristics of the patient.
20. The system of any one of clauses 14 to 19, wherein the sleep data comprises REM, light sleep state, or deep sleep state.
21. The system of clause 20, wherein the user interface displays the sleep data and time the patient spent in REM, light sleep state, or deep sleep state.
22. The system of either clause 20 or clause 21, wherein the user interface displays time the patient spent sleeping, being in in bed, and being out of bed.
23. The system of any one of clauses 14 to 22, wherein the sleep status of the patient is projected on the floor.
24. A method of displaying movement data collected from a support apparatus comprising an inflatable mattress having multiple inflatable zones, the method comprising the steps of:
   monitoring signals from a plurality of apparatus component sensors,
   positioning the mattress on a physiological sensor,
   monitoring sleep signals from the physiological sensor,
   using a controller to process the signals from the load cells, pressure sensors, and the physiological sensor to identify movement data and patient sleep data, and
   displaying the movement data and the sleep data of a patient on the mattress on a segmented display on a user interface.
25. The method of clause 24, wherein the method comprises automatically moving a portion of the support apparatus to mitigate a future event based on the movement data or the sleep data of the patient.
26. The method of clause 25, wherein the future event is sleep apnea.
27. The method of any one of clauses 24 to 26, wherein the method comprises changing inflation of one of the multiple inflatable zones of the inflatable mattress based on the movement data or the sleep data of the patient.
28. The method of any one of clauses 24 to 27, wherein the movement data comprises patient motion data and non-patient motion artifacts.
29. The method of any one of clauses 24 to 28, wherein the movement data comprises upper torso movement and lower body movement.
30. The method of any one of clauses 24 to 29, wherein the physiological sensor is operable to detect physiological characteristics of the patient.
31. The method of any one of clauses 24 to 30, wherein the sleep data comprises REM, light sleep state, or deep sleep state.
32. The method of any one of clauses 24 to 31, wherein the sleep status of the patient is projected on the floor.
33. A patient support apparatus comprising:
   a patient support surface;
   a siderail located adjacent the patient support surface;
   a headboard located adjacent the patient support surface;
   a footboard located adjacent the patient support surface;
   a frame supporting the patent support surface;
   at least one sensor supported by the frame and located lower than the patient support surface, wherein the sensor is operable to generate, without contacting the patient, signals indicative of at least one of a heart rate, respiration rate, and movement of a patient supported on the support surface;
   a controller operable to process the signals to determine at least one of movement data and sleep data of a patient;
   a user interface held to at least one of the siderail, headboard, footboard, frame, and sensor, the user interface illustrating at least one of a movement condition generated from the movement data and a sleep condition generated from the sleep data.
34. The patient support apparatus as recited in clause 33, wherein the user interface comprises at least one of an indicator light and a projection light.
35. The patient support apparatus as recited in either clause 33 or clause 34, wherein the user interface comprises a touchscreen display held to at least one of the siderail, headboard, and footboard.
36. The patient support apparatus as recited in any one of clauses 33 to 35, wherein the user interface comprises an integrated light held within at least one of the siderail, headboard, footboard, and frame.

## Claims

1. A patient support apparatus comprising:
a plurality of apparatus component sensors,
a physiological sensor,
a user interface including a segmented display, the segmented display illustrating movement data and sleep state data of a patient, and
a control system including a controller in communication with the plurality of bed component sensors and the physiological sensor, the controller operable to receive a separate signal from each of the apparatus component sensors to monitor movement detected by the apparatus component sensors, a sleep signal from the physiological sensor, and
wherein the controller is further operable to process the signals to determine the movement data and sleep data to detect a sleep event and alter a portion of the patient support apparatus to mitigate the sleep event.

2. The patient support apparatus of claim 1, wherein the controller moves a head section of the patient support apparatus in response to the sleep event.

3. The patient support apparatus of either claim 1 or claim 2, wherein the segmented display comprises a first segment including an icon for each day of a week, and wherein the activation of the icon for a particular day results in an illustration of the movement data or the sleep data for that day in a second segment.

4. The patient support apparatus of any preceding claim, wherein the patient support apparatus comprises a siderail having an illuminated grip and the sleep state of the patient is indicated by illuminating the grip using a specific scheme to indicate the sleep state.

5. The patient support apparatus of either claim 3 or claim 4, wherein the second segment displays the movement data, and wherein the movement data comprises upper torso movement and lower body movement.

6. The patient support apparatus of any ones of claims 3 to 5, wherein the second segment displays the movement data, and wherein the movement data comprises time the patient spent moving and not moving over a period of one week.

7. The patient support apparatus of any one of claims 3 to 6, wherein the second segment displays the movement data, and wherein the movement data comprises percentage of time the patient spent in upper torso movement and lower body movement over a period of one week.

8. The patient support apparatus of any one of claims 3 to 7, wherein the second segment displays the sleep data, and wherein the sleep data comprises REM, light sleep state, or deep sleep state.

9. The patient support apparatus of any one of claims 3 to 8, wherein the second segment displays the sleep data, and wherein the sleep data comprises time the patient spent sleeping, being in bed, or being out of bed.

10. The patient support apparatus of any one of claims 3 to 9, wherein the second segment displays the sleep data, and wherein the sleep data comprises time the patient spent in REM, light sleep state, or deep sleep state over a period of one week.

11. The patient support apparatus of any one of claims 3 to 10, wherein the second segment displays the sleep data, and wherein the sleep data comprises time the patient spent sleeping, being in in bed or being out of bed over a period of one week.

12. The patient support apparatus of any preceding claim, wherein the physiological sensor is operable to detect the respiration rate or heart rate of the patient.

13. The patient support apparatus of any preceding claim, wherein the sleep status of the patient is projected on the floor.

14. The patient support apparatus of any preceding claim , wherein the sleep event is a sleep apnea event.
